# EUROPÄISCHE PATENTSCHRIFT

(11) **EP 1 326 964 B1**
(45) Veröffentlichungstag und Bekanntmachung des Hinweises auf die Patenterteilung: **19.01.2011**
(21) Anmeldenummer: 01983527.1
(22) Anmeldetag: 05.10.2001
(51) Int. Cl.: C12N 9/12

(54) **THERMOSTABILE POLYMERASE AUS i THERMOCOCCUS PACIFICUS /i**
THERMOSTABLE POLYMERASE BASED ON i THERMOCOCCUS PACIFICUS /i
POLYMERASE THERMOSTABLE A BASE DE i THERMOCOCCUS PACIFICUS /i

(30) Priorität: 05.10.2000 DE 10049211
(43) Veröffentlichungstag der Anmeldung: 16.07.2003
(73) Patentinhaber: QIAGEN GmbH, 40724 Hilden (DE)
(72) Erfinder: DECKER, Katja, 40699 Erkrath (DE); LÖFFERT, Dirk, 40589 Düsseldorf (DE); KANG, Jie, 40822 Mettmann (DE)
(74) Vertreter: Kilger, Christian
(86) Internationale Anmeldenummer: PCT/EP2001/011529
(87) Internationale Veröffentlichungsnummer: WO 2002/029016

(56) Entgegenhaltungen:
- EP-A- 0 745 675
- WO-A-01/61015
- WO-A-98/14590
- WO-A-98/49274
- DATABASE EMBL [Online] KOMATSUBARA H. ET AL.: "Thermally Stable DNA Polymerase Derived from Thermococcus Peptonophilus, Gene Encoding the Same Enzyme and Use Therfore. " retrieved from EBI Database accession no. E13952 XP002192769
- HOPFNER KARL-PETER ET AL: "Crystal structure of a thermostable type B DNA polymerase from Thermococcus gorgonarius" PROCEEDINGS OF THE NATIONAL ACADEMY OF SCIENCES OF USA, NATIONAL ACADEMY OF SCIENCE. WASHINGTON, US, Bd. 96, Nr. 7, 30. März 1999 (1999-03-30), Seiten 3600-3605, XP002164083 ISSN: 0027-8424

## Beschreibung

Die vorliegende Erfindung betrifft eine thermostabile Polymerase aus *Thermococcus pacificus,* DNA-Moleküle, die für eine solche Polymerase kodieren, Expressionsvektoren, Wirtszellen, Verfahren zur Herstellung einer solchen Polymerase und deren Verwendung zur Polymerisation von Nukleinsäure, insbesondere In der Polymerase-Kettenreaktion.

DNA-Polymerasen sind eine Familie von Enzymen, die die Polymerisation von Nukleinsäuren katalysieren und eine Rolle sowohl bei der DNA-Replikation als auch bei der DNA-Reparatur spielen. Thermostabile DNA-Polymerasen werden häufig in in-vitro-Verfahren verwendet, zum Beispiel bei der Polymerase-Kettenreaktion (PCR), die ein unverzichtbares Verfahren der Molekularbiologie geworden ist. Ein gemeinsame Problem der dafür verwendeten DNA-Polymerasen ist der Einbau falscher Nukleotide während der DNA-Synthese, der zu mutierten PCR-Produkten führt. Dies kann in bestimmten molekularbiologischen Anwendungen zu Problemen führen, Insbesondere bei der Klonierung und anschließenden rekombinanten Expression von Protein, da die eingeführten Mutationen zu inaktivem Protein oder Protein führen können, das in seinen Eigenschaften vom ursprünglichen Protein abweicht. Der Fehleinbau kann durch Polymerasen korrigiert werden, die eine inhärente 3'-5'-Exonuklease-Aktivität aufweisen (sog. Proofreading-Enzyme). Das in der PCR meistverwendete Enzym, Taq DNA-Polymerase, weist eine solche enzymatische Aktivität nicht auf, und es ist bekannt, daß die Fehlerrate dieses Enzyms mehr als zehnmal höher ist als die der Proofreading-Enzyme (US 5,545,552).

Verschiedene andere bekannte thermostabile DNA-Polymerasen weisen zwar Proofreading-Aktivität auf, sind aber mit anderen Nachteilen behaftet (Lundberg et al. 1991, Gene 108:1-6; EP 0 455 430; EP 0 701 000; WO 92/03556; WO 92/09689). Thermostabile DNA-Polymerasen mit 3'-5'-Exonuklease-Aktivität sind Insbesondere auch aus den Organismen *Thermococcus gorgonarius* (WO 98/14590 A1) und dem Archaeon-Stamm KOD1 (EP 0 745 675 A2) bekannt.

Es besteht weiterhin Bedarf nach neuen thermostabilen DNA-Polymerasen mit Proofreading-Aktivität und im Hinblick auf ihre Verwendbarkeit in der Molekularbiologie verbesserten Eigenschaften, insbesondere erhöhte Thermostabilität, 3'-5'-Exonuklease-Aktivität, und Fehlerkorrekturiesefähigkeit unter PCR-Bedingungen.

Gelöst wird diese Aufgabe durch die Bereitstellung einer neuen thermostabilen DNA-Polymerase, erhältlich aus dem Organismus *Thermococcus pacificus.*

Die vorliegende Erfindung betrifft insbesondere eine thermostabile DNA-Polymerase aus *Thermococcus pacificus* mit 3'-5'-Exonuklease-Aktivität. Vorzugsweise hat eine solche DNA-Polymerase die Aminosäuresequenz SEQ ID NO: 2 (numerische Kennzahl <210> 2 bzw. <400> 2 im anliegenden Sequenzprotokoll).

In einem weiteren Aspekt betrifft die vorliegende Erfindung ein DNA-Molekül, das für eine thermostabile DNA-Polymerase mit 5'-3'-Polymerase-Aktivität kodiert, und das
(a) die Sequenz SEQ ID NO: 1 (numerische Kennzahl <210> 1 bzw. <400> 1 im anliegenden Sequenzprotokoll) oder die dazu komplementäre Sequenz enthält; oder
(b) eine Sequenz enthält, die für einen zusammenhängenden Abschnitt von wenigstens 200 Aminosäuren der Sequenz SEQ ID NO: 2 kodiert, oder
(c) eine Sequenz enthält, die der SEQ ID NO: 1 oder der dazu komplementären Sequenz so ähnlich ist, dass das DNA Molekül unter stringenten Bedingungen, bei denen zwei DNA-Moleküle in einem Hybridisieriengsexperiment hybridisieren, wenn sie über die gesamte Länge eine Sequenzidentität von 98% oder mehr aufweisen, miteinander hybridisieren,
(e) mit dem Proviso, das nicht das Polynukleotid umfasst ist, welches für eine Polymerase kodiert, bestehend aus (i) Aminosäuren 271 bis 832 von *Taq* Polymerase (SEQ ID NO. 7 in WO 01/61015), verbunden mit (ii) Aminosäuren 1 bis 395 von *Tpac* (SEQ ID NO. 16 in WO 01/61015).

Unter stringenten Bedingungen sollen im Sinne der vorliegenden Erfindung Bedingungen zu verstehen sein, unter denen zwei DNA-Moleküle in einem Hybridisierungsexperiment miteinander hybridisieren, wenn sie im hybridisierenden Abschnitt eine Sequenzidentität von 97 % oder mehr aufweisen, vorzugsweise 98% oder mehr, besonders bevorzugt 99% oder mehr. Dem Fachmann ist bekannt, wie er solche Bedingungen einstellen kann (Sambrook, Fritsch, Maniatis. Molecular Cloning. A Laboratory Manual, 1989. 9.47).

Ein Weg, ein erfindungsgemäßes DNA-Molekül zu erhalten, besteht darin, es aus dem Organismus *Thermococcus pacificus* zu isolieren. *Thermococcus pacificus* ist im Stand der Technik bekannt (Miroshnichenko et al. 1998, Thermococcus gorgonarius sp. nov. and Thermococcus pacificus sp. nov.: heterotrophic extremely thermophilic archaea from New Zealand submarine hot vents. Int. J. Syst. Bacteriol. 48:23-29) und aus öffentlichen Sammlungen erhältlich (DSMZ-Deutsche Sammlung von Mikroorganismen und Zellkulturen GmbH, Mascheroder Weg 1 b, 38124 Braunschweig, Deutschland, DSM No. 10394; American Type Culture Collection, 10801 University Boulevard, Manassas, VA 20910, USA, ATCC 700653).

Der Organismus kann in an sich bekannter Weise angezüchtet werden. Dabei kann ein Fertigmedium der Fa. Becton Dickinson (Bacto Marine Broth 2216) verwendet werden. Das Ansetzen des Mediums kann nach Angaben des Herstellers sowie der DSMZ (Medien 514 und 760) erfolgen; dabei kann unter geringem Verlust der Ausbeute auf die Zugabe von Schwefel verzichtet werden. Das Medium kann durch Begasen mit N₂ anaerobisiert werden; dies kann aufgrund des Zusatzes von Resazurin (1 mg/l Endkonzentration) überprüft werden. Um beispielsweise größere Kulturvolumina anzuzüchten, können zunächst Vorkulturen angesetzt werden. Dazu können 2 ml der von der DSMZ erhaltenen Ausgangskultur (DSM 10394) unter anaeroben Bedingungen in 20 ml Medium überimpft werden. Die Inkubation kann über Nacht bei 85 °C ohne Schütteln in luftdicht verschlossenen Kulturflaschen erfolgen. Zum Ansetzen der Hauptkulturen können 20 ml der Vorkultur in 500 ml frisches Medium anaerob überimpft und unter gleichen Bedingungen inkubiert werden wie die Vorkulturen.

Das Ernten der Kulturen kann durch Überführen der Kulturen in anaerobisierte Zentrifugenbecher und anschließende Zentrifugation der Proben für 15 min bei 4°C und 4000 x g erfolgen. Das so erhaltene Zellpellet kann dann für die Präparation der genomischen DNA nach gängigen Methoden verwendet werden.

Die gereinigte genomische DNA kann einer PCR-Reaktion beispielsweise mit den Primern SEQ ID NO. 3, ATGATCCTCGATGCCGACTAC (Tpac3') und SEQ ID NO. 4, TCATGTCTTAGGTTTTAGCCACGC (Tpac5') unterworfen werden, bei der die SEQ ID NO. 1 amplifiziert wird. Das Amplifikationsprodukt kann dann nach Standardverfahren aufgereinigt werden, z.B. mit dem QIAquick PCR Purification Kit oder QIAquick Gel Extraction Kit (QIAGEN GmbH, Hilden, Germany).

Ein anderer Weg, ein DNA-Molekül der vorliegenden Erfindung herzustellen, besteht In der chemischen Synthese eines solchen DNA-Moleküls. Dafür können beispielsweise zunächst geeignete Oligonukleotide nach an sich bekannten Verfahren der Oligonukleotidsynthese hergestellt werden (z.B. Gait,M.J., 1984, Oligo*nucleotide Synthesis. A Practical Approach*.IRL Press, Oxford, UK), aus denen dann nach verschiedenen Methoden ein synthetisches Gen hergestellt werden kann. Solche Verfahren sind im Stand der Technik bekannt (z.B. Stemmer et al. 1995, Single-step assembly of a gene and entire plasmid from large numbers of oligodeoxyribonucleotides, Gene 164(1): 49-53; Ye et al. 1992, Gene synthesis and expression in E. coli for pump, a human matrix metalloproteinase, Biochem Biophys Res Commun 186(1):143-9; Hayden et Mandecki 1988, Gene synthesis by serial cloning of ollgonucleotides, DNA 7(8): 571-7). Auf diese Welse kann beispielsweise ein DNA-Molekül mit der Sequenz SEQ ID NO: 1 nach an sich bekannten Methoden hergestellt werden.

Es liegt im Können des Durchschnittsfachmanns, DNA-Moleküle herzustellen, die Varianten eines DNA-Moleküls mit der SEQ ID NO: 1 darstellen und gleichwohl für eine thermostabile DNA-Polymerase mit im wesentlichen unveränderten Eigenschaften kodieren. Solche Varianten unterscheiden sich von DNA-Molekülen mit der SEQ ID NO: 1 dadurch, daß ein, zwei, drei, vier, fünf, sechs, sieben, acht, neun, zehn oder mehr Nukleotide deletiert oder durch Nukleotide mit anderen Basen substituiert sind, oder zusätzliche Nukleotide inseriert oder addiert sind. Durch Routineexperimente kann der Fachmann feststellen, ob eine solche Modifikation einen signifikanten Einfluß auf die Eigenschaften des durch ein solches DNA-Molekül kodierten Polypeptids hat. Er hat keinerlei Schwierigkeiten, durch Routineexperimente eine große Zahl solcher Varianten aufzufinden, die für eine thermostabile DNA-Polymerase mit im wesentlichen unveränderten Eigenschaften kodieren. Solche Varianten sind daher ausdrücklich in die Erfindung mit eingeschlossen.

So kann der Fachmann insbesondere Varianten herstellen, die gegenüber einem DNA-Molekül mit der Sequenz SEQ ID NO: 1 im Hinblick auf den genetischen Code degeneriert sind, d.h. DNA-Moleküle mit anderer Nukleotidsequenz als SEQ ID NO: 1, die aber für die gleiche Aminosäuresequenz kodieren. Dies kann sinnvoll sein, um bei der Expression eines solchen DNA-Moleküls in einer Wirtszelle die Codons im Hinblick auf die bevorzugt verwendeten Codons der betreffenden Wirtszelle zu optimieren. Dem Fachmann sind entsprechende Verfahren bekannt.

Es liegt ferner im Können des Durchschnittsfachmanns, kleinere oder größere Sequenzabschnitte zu deletieren, was bei der Expression zu Polypeptiden mit entsprechenden Deletionen in der Aminosäuresequenz führt. Durch an sich bekannte Computerverfahren, die auf Vergleichen einer Sequenz, im vorliegenden Fall SEQ ID NO: 1, mit den Sequenzen anderer und gut charakterisierter Proteine beruhen, kann der Fachmann feststellen, welche Sequenzabschnitte (Domänen) für die enzymatischen Aktivitäten, Polymerase- bzw. Exonuklease-Aktivität, verantwortlich sind. Insbesondere außerhalb dieser Domänen sind Deletionen (und natürlich auch Substitutionen) möglich, die ohne wesentlichen Einfluß auf die enzymatische Aktivität bleiben. Ob eine bestimmte Deletion in diesem Sinne ohne Auswirkungen bleibt, ist leicht feststellbar.

Es ist ferner möglich, das DNA-Molekül so zu modifizieren, daß die erfindungsgemäße DNA-Polymerase als Fusionsprotein mit zusätzlichen Aminosäuresequenzen vorliegt. So kann z.B. an den C-Terminus ein Affinitätsmarker in Form einer kurzen Peptidsequenz angefügt werden, beispielsweise ein Histidin-Hexamer, der die Reinigung des Proteins erleichtert (siehe unten).

Der Fachmann kann auch gezielt eine der belden enzymatischen Aktivitäten durch Mutation ausschalten, beispielsweise durch Substitution oder Deletion einer oder mehrerer Aminosäuren, oder durch Deletion eines ganzen Sequenzabschnitts inder entsprechenden Domäne bis hin zur Deletion der ganzen Domäne. So kann der Fachmann beispielsweise ein DNA-Molekül herstellen, das für ein Polypeptid kodiert, das eine Polymerase-, nicht jedoch eine Exonuklease-Aktivität aufweist. Dies kann vorteilhaft sein, wenn ein solches Polypeptid beispielsweise in der DNA-Sequenzierung Anwendung finden soll.

In einer besonderen Ausführungsform kodiert das erfindungsgemäße DNA-Molekül für ein Polypeptid, das sowohl 5'-3'-DNA-Polymerase- als auch 3'-5'-Exonuklease-Aktivität hat.

Ein weiterer Aspekt der vorliegenden Erfindung ist ein Vektor, der ein erfindungs gemäßes DNA-Molekül enthält, insbesondere ein Expressionsvektor. Vorzugsweise weist ein solcher Expressionsvektor die folgenden Merkmale auf:
(a) einen oder mehrere Promotoren;
(b) mindestens einen Operator, der zu Steigerung oder Unterdrückung der Genexpression verwendet werden kann;
(c) Terminationssequenzen für Transkription und Translation.

In einem solchen Expressionsvektor steht das erfindungsgemäße DNA-Molekül im operativen Zusammenhang mit diesen Merkmalen, so daß ein solcher Expressionsvektor die Expression der erfindungsgemäßen thermostabilen DNA-Polymerase in einer Wirtszelle erlaubt. Expressionsvektoren, die für solche Zwekke geeignet sind, sind im Stand der Technik in großer Zahl bekannt, ebenso Verfahren zum Einbringen des erfindungsgemäßen DNA-Moleküls in einen solchen Vektor, Einbringen des Vektors in Wirtszellen, Kultivierung der Wirtszellen und Isolierung des gebildeten Polypeptids (s. z.B. Sambrook et al., Molecular Cloning, 2nd Ed., Cold Spring Harbour 1989, insbesondere Kapitel 17). Geeignete Expressionsvektoren sind beispielsweise die pQE-Vektoren, die über die Firma Qiagen - GmbH, 40724 Hilden, Deutschland kommerziell erhältlich sind. Diese Vektoren erlauben gleichzeitig den Einbau eines Affinitätsmarkers, beispielsweise eines Histidin-Hexamers, in das gebildete Polypeptid, mit dessen Hilfe eine einfache und effektive Aufreinigung des Polypeptids möglich ist (Crowe et al., 1994, 6xHis-Ni-NTA chromatography as a superior technique in recombinant protein expression/purification, Methods Mol Biol. 31:371-87; Stüber et al. 1990, System for highlevel production in Escherichia coli and rapid purification of recombinant proteins, Immunol. Methods 4:121).

Entsprechend ist ein weiterer Aspekt der vorliegenden Erfindung eine Wirtszelle, die solch einen Vektor enthält. Bevorzugt ist dabei Escherichia coli als Wirtszelle. Ein weiterer Aspekt der vorliegenden Erfindung ist ein Verfahren zur Herstellung der erfindungsgemäßen DNA-Polymerase, dadurch gekennzeichnet, daß (a) wie vorstehend beschriebene Wirtszellen in einem geeigneten Medium kultiviert werden; und (b) das gebildete Polypeptid aus dem Medium oder den Wirtszellen isoliert wird.

Ein weiterer Aspekt der vorliegenden Erfindung ist ein Polypeptid, das durch Expression eines erfindungsgemäßen DNA-Moleküls hergestellt werden kann. Insbesondere ist dies ein Polypeptid mit 5'-3'-DNA-Polymerase-Aktivität, vorzugsweise zusätzlich mit 3'-5'-Exonuklease-Aktivität.

Ferner ist ein Polypeptid Gegenstand der vorliegenden Erfindung, das DNA-Polymerase-Aktivität hat, vorzugsweise 5'-3'-DNA-Polymerase-Aktivität, und eine Sequenz enthält, die einen zusammenhängenden Abschnitt von wenigstens 200 Aminosäure der Sequenz SEQ ID NO: 2 darstellt. Besonders bevorzugt ist ein Polypeptid mit der Sequenz SEQ ID NO: 2 oder ein Polypeptid, das die Sequenz SEQ ID NO: 2 enthält.

Die erfindungsgemäße thermostabilen DNA-Polymerase kann vorteilhaft zur Polymerisation von Nukleinsäure eingesetzt werden, insbesondere bei der Polymerase-Kettenreaktion (PCR). Sie ist sehr thermustabli, effizient und weist durch ihre - Proofreading-Aktivität nur einen sehr geringen Fehleinbau auf. Verfahren zur Polymerisation von Nukleinsäure durch matrixabhängige Polymerisation von Nukleotiden unter Verwendung von DNA-Polymerasen als Katalysatoren, insbesondere die Polymerase-Kettenreaktion, sind im Stand der Technik bekannt (s. z.B. Sambrook et al., Molecular Cloning, 2nd Ed., Cold Spring Harbour 1989, insbesondere Kapitel 14).

Ein weiterer Aspekt der vorliegenden Erfindung ist ein Kit zur Verwendung für die Polymerisation von Nukleinsäure, in separaten Behältern enthaltend
(a) ein Polypeptid gemäß der Erfindung; und
(b) einen Reaktionspuffer für die Polymerisationsreaktion.

Optional kann ein solches Kit zusätzlich dATP, dGTP, dCTP, und dTTP enthalten, entweder als Gemisch oder in jeweils individuellen Behältern.

### Abbildungen

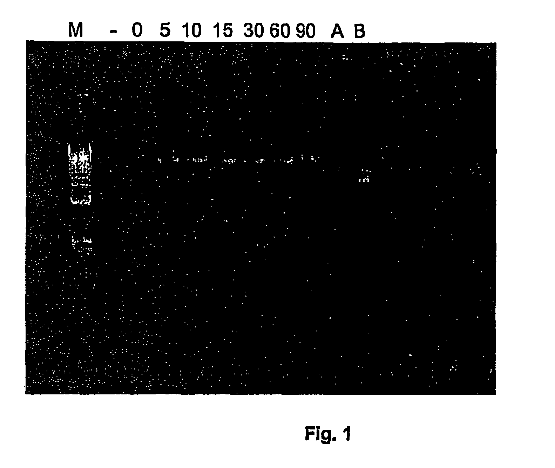

**Abbildung 1**: Thermostabilität der *Thermococcus pacificus* DNA Polymerase. Siehe Beispiel 6. M: Marker; - : Leere Spur; 0-90: Präinkubation der Polymerase für 0, 5, 10 usw. Minuten bei 95°C; A, B: M13 DNA Einzelstrang-Kontrollen ohne Zusatz von Polymerase

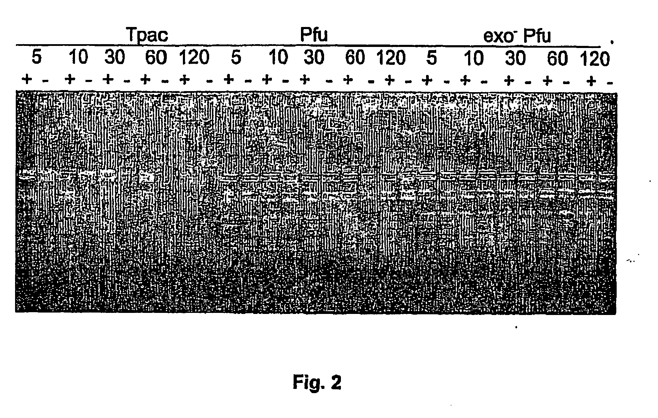

**Abbildung 2**: Nachweis der 3'-5' Exonukleaseaktivität der *Thermococcus pacificus* DNA Polymerase. Siehe Beispiel 7. Tpac: *Thermococcus pacificus* Polymerase; Pfu: *Pyrococcus furiosus* Polymerase; exo Pfu: *Pyrococcus furiosus* Polymerase, deren 3'-5' Exonuklease mutiert ist, so dass keine Exonuklease-Aktivität nachweisbar ist; +: Zugabe von dNTPs; -: Reaktionsansatz ohne dNTPs; 5, 10...: Inkubationsdauer in Minuten.

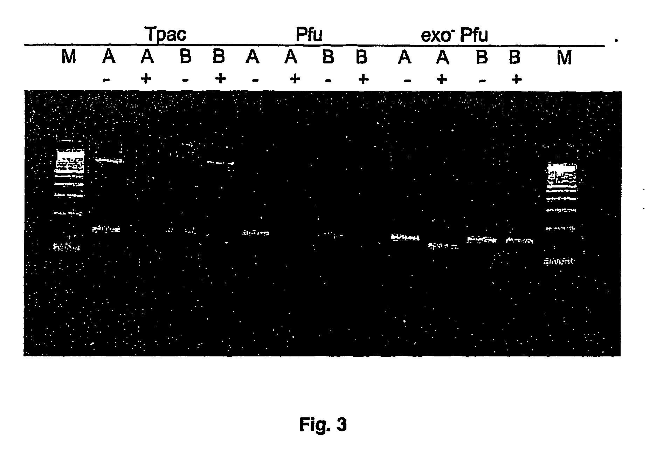

**Abbildung 3**: Nachweis der Fehlerkorrekturlesefähigkeit *der Thermococcus pacificus* DNA Polymerase unter PCR Bedingungen. Siehe Beispiel 8. Tpac: *Thermococcus pacificus* Polymerase; Pfu: *Pyrococcus furiosus* Polymerase; exo⁻ Pfu: *Pyrococcus furiosus* Polymerase, deren 3'-5' Exonuklease mutiert ist, so dass keine Exonuklease-Aktivität nachweisbar ist; A, B: kennzeichnen 2 voneinander unabhängige PCR Reaktionen; M: Marker; +: Verdau mit BamHI erfolgt; -: nicht verdautes PCR Fragment

### Beispiele

### Beispiel 1: Kultur von Thermococcus pacificus und isolierung von genomischer DNA

Der Organismus DSM No. 10394 wurde unter Verwendung eines Fertigmediums der Fa. Becton Dickinson (Bacto Marine Broth 2216) kultiviert. Das Ansetzen des Mediums erfolgte nach Angaben des Herstellers sowie der DSMZ (Medien 514 und 760) unter Verzicht auf Schwefel. Das Medium wurde durch Begasen mit N₂ anaerobisiert; dies konnte durch Zusatz von Resazurin (1 mg/l Endkonzentration) überprüft werden.

Zunächst wurden Vorkulturen angesetzt. Dazu wurden 2 ml der von der DSMZ erhaltenen Ausgangskultur (DSM 10394) unter anaeroben Bedingungen in 20 ml Medium überimpft. Die Inkubation erfolgte über Nacht bei 85 °C ohne Schütteln in luftdicht verschlossenen Kulturflaschen. Zum Ansetzen der Hauptkulturen wurden 20 ml der Vorkultur in 500 ml frisches Medium anaerob überimpft und unter gleichen Bedingungen inkubiert wie die Vorkulturen.

Das Ernten der Kulturen erfolgte durch Überführen der Kulturen in anaerobisierte Zentrifugenbecher und anschließende Zentrifugation der Proben für 15 min bei 4°C und 4000 x g. Das so erhaltene Zellpellet wurde dann für die Präparation der genomischen DNA unter Verwendung eines kommerziell erhältlichen Reinigungskits (Qiagen Genomic-tip System, QIagen GmbH, Hilden, Deutschland) gemäß Herstellerangaben verwendet.

### Beispiel 2: Herstellung eines Polymerase-Expressionsvektors

Der Expressionsvektor wurde mit Hilfe allgemein bekannter molekularbiologischer Methoden hergestellt. Das Polymerasegen wurde mittels Polymerase-Ketten-Reaktion (PCR) aus der genomischen DNA des Organismus *Thermococcus pacificus* isoliert. Die hierzu verwendeten Primer enthielten neben der zum Polymerasegen homologen Sequenzen zusätzlich jeweils eine nicht komplementäre Nukleinsäuresequenz, die für eine Restriktionsschnittstelle kodierte, so daß durch eine Restriktion des Amplifikats und des Expressionsvektors das Amplifikat in den Expressionsvektor kloniert werden kann. Oligonukleotide wurden von Life Technologies GmbH, Karlsruhe, Deutschland, bezogen. Weitere Reagenzien zur Durchführung der Polymerase-Ketten-Reaktion wie *Taq* DNA Polymerase wurden von QIAGEN GmbH, Hilden, Deutschland bezogen. Die kodierende Polymerasegensequenz SEQ ID NO: 1 wurde unter Verwendung von 2.5 units *Taq* DNA Polymerase oder der Proofreading DNA Polymerase *Pfu* DNA Polymerase (Stratagene, Heidelberg, Deutschland) amplifiziert. Weitere Reaktionszusätze waren Oligonukleotide (0.2-1.0 µM) als Primer, 200 µM von jedem dNTP und 1x Reaktionspuffer der entsprechenden Polymerase. Es wurde ein 3-Schritt PCR Programm bestehend aus Denaturierungsschritt zum Aufschmelzen der Ausgangsnukleinsäure bei ca. 94°C, einem Aniagerungsschritt der Oligonukleotide an Ihre komplementäre DNA Sequenz bei ca. 50-68°C und einem Extensionsschritt bei ca. 72°C zur Amplifikation durchgeführt. In Abhängigkeit von der Ausgangsnukleinsäuremenge wurden 30 bis 40 Amplifikationszyklen ausgeführt. Nach Durchführung der PCR Reaktion wurde das Reaktionsprodukt auf einem Agarosegel im Vergleich mit einem geeigneten DNA Größenmarker auf seine spezifische Länge überprüft. PCR Produkte, die die erwartete Größe aufwiesen, wurden entweder direkt aus dem Gel oder aus der PCR Reaktion aufgereinigt. Hierzu wurden kommerziell erhältliche Systeme verwendet (QIAquick PCR Purification Kit oder QIAquick Gel Purification Kit, QIAGEN GmbH, Hilden, Deutschland). Aufgereinigtes PCR Produkt und Vektor-DNA wurden mit den entsprechenden Restriktionsenzymen geschnitten und die Reaktionsprodukte wiederum wie oben beschrieben aufgereinigt. Als Vektor-DNA wurde das Plasmid pQE80 eingesetzt (QIAGEN GmbH, Hilden), das nach Einfügen des Zielgens ein Fusionsprotein aus einem sogenannten His-Tag und dem Zielprotein exprimieren kann. In der folgenden Ligationsreaktion wurden equimolare Mengen Vektor-DNA und PCR Produkt eingesetzt und unter Verwendung von T4-Ligase (Life Technologies GmbH, Karisruhe, Deutschland), dem entsprechenden Reaktionspuffer und ATP über Nacht in einem Endvolumen von 20 µl bei ca. 16°C ligiert.

### Beispiel 3: Herstellung einer Thermococcus pacificus DNA Polymerase exprimierenden Bakterienzelle

1 bis 2 µl der Ligationsreaktion wurden in Calcium-kompetente DH5α-Bakterienzellen transformiert, die optionsweise zusätzlich das Plasmid pRep4 (QIAGEN GmbH, Hilden) enthielten. Ein Teil der Transformationsreaktion wurde anschließend auf eine Agarplatte ausplattiert, die das Antibiotikum Ampicillin und Kanamycin oder nur Ampicillin als Selektionsmarker enthielten. Die Platten wurden über Nacht für ca. 15 bis 18 Stunden bei 37°C inkubiert. Anschließend wurden Bakterienkolonien mit sterilen Zahnstochern oder Pipettenspitzen gepickt, in ca. 3 ml LB-Medium mit dem geeigneten Antibiotikum überführt und über Nacht bei 37°C inkubiert. Plasmide wurden am nächsten Tag nach Herstellerangaben mit kommerziell erhältlichen Kits wie dem QIAprep Mini Kit oder den QIAGEN Plasmid Tips isoliert (QIAGEN GmbH, Hilden, Deutschland). Plasmide wurden anschließend mit geeigneten Restriktionsenzymen und Sequenzierung darauf hin überprüft, ob sie das Polymerasegen enthielten.

### Beispiel 4: Expression und Reinigung der DNA Polymerase von Thermococcus pacificus

Ein Konstrukt, welches die fehlerfreie Nukleinsäure-sequenz der DNA Polymerase enthielt, wurde in DH5α/pRep4 kompetente Zellen transformiert. Zellen wurden in Anwesenheit von Ampicillin und Kanamycin in NZ-Amine Medium kultivert und die Expression des Polymerasegens durch Zugabe von IPTG induziert. Nach der Ernte der Bakterienzellen, wurden die Bakterienzellen mit Lysozym, Ultraschall und kurzem Aufkochen aufgeschlossen. Das mit einem His-Tag versehene Polymeraseproteln wurde unter Verwendung eines kommerziell erhältlichen Reinigungskits (QIAexpress Protein Purification System, QIAGEN GmbH, Hilden, Deutschland) nach Herstellerangaben mittels Metall-Affinitätschromatographie mit Nickel-NTA-Agarose selektiv aufgereinigt. Das mit Imidazol eluierte Protein wurde gegen eine Lagerungspuffer dialysiert, der aus 20 mM TrisHCl (pH 8 bei 20°C), 100 mM KCI, 1 mM EDTA, 0.5% (v/v) Nonidet P-40 Ersatz, 0.5% (v/v) Tween 20 und 50% (v/v) Glycerin bestand. Die Polymerase wurde In diesem Puffer bei - 20°C gelagert.

### Beispiel 5: 5'-3' Polymeraseaktivität der Thermococcus pacificus DNA Polymerase

Um nachzuweisen, daß die kionierte Nukleinsäuresequenz für eine DNA Polymerase kodiert, wurde ein Test zur Überprüfung auf eine DNA Polymeraseaktivität durchgeführt. Der Assay weist die Extension eines Oligonukleotids nach, das an einzelsträngige M13-DNA hybridisiert vorliegt. Wird der Primer verlängert, was nur geschehen kann, wenn die zugesetzte Proteinpräparation eine DNA Polymeraseaktivität aufweist, wird aus der einzelsträngigen Ausgangsnukleinsäure ein dop-pelsträngiges DNA Molekül gebildet. Die Aktivität wird anschließend auf einem Agarosegel anhand eines Wanderungsunterschieds der doppelsträngigen DNA im Unterschied zur einzelsträngigen Ausgangs-DNA nachgewiesen. Hierbei ist die Extensionsrate abhängig von der verwendeten Polymerase. Die Endproduktmenge an doppelsträngiger DNA ist abhängig von der Menge an DNA Polymerase, die polymerasenspezifische Extensionsrate und der Zelt, die zur Durchführung der Reaktion ausgewählt wurde.

Alle Poiymerisatfonsreaktionen enthielten 50 ng M13mp18-DNA (20 fmol; 7250 Basen), 0.1 µM 30-mer Oligonucleotid der Sequenz 5'-TTTCCCAGTCACGACGTTGTAAAACGACGG-3' (SEQ ID NO: 5) und 50 µM von jedem dNTP in 10 µl 10 mM TrisHCl. Polymerisationsreaktionen enthielten verschiedene Mengen an *Taq* DNA Polymerase (0.2, 0.1, 0.05 und 0.01 units; QIAGEN GmbH, Hilden, Deutschland) oder die zu testende Polymerasepräparation in verschiedenen Verdünnungen. Die Reaktion wurde für beide Enzyme in 1x Reaktionspuffer der *Taq* DNA Polymerase (QIAGEN GmbH, Hilden) durchgeführt, dem 1 µg/ml BSA zugesetzt wurde, um unspezifische Proteinbindestellen auf der Oberfläche des Reaktionsgefäßes abzusättigen.

Die Polymerisationsreaktion wurde in einem Thermocycler von MJ Research (Biozym, Hess. Oldendorf, Deutschland) des Modells PTC-200 durchgeführt. Die Reaktionsbedingungen wurden wie folgt gewählt: 1 sec Denaturierung, um eventuell vorhandene Sekundärstrukturen In der DNA aufzulösen, 30 sec Hybridisierung des Oligonukleotids bei 55°C, die von der Primerextension bei 72°C für 3 min gefolgt wurde.

Nach Abschluß der Reaktionen wurden die Reaktionsprodukte mit 1 µl Gelladepuffer vermengt (50% Glycerin, 1x TAE Puffer, 0.02 mg/ml Bromphenolblau) und auf ein 1%iges Agarosegel aufgetragen, das zur Anfärbung der DNA 0.5 µg/ml Ethidiumbromid enthielt. Das Gel wurde bei 80 mA für ca. 15 min in 1x TAE Puffer analysiert, um eine Auftrennung von einzelsträngiger und doppelsträngiger DNA zu erreichen. Die Ergebnisse zeigen, daß die Proteinpräparation, die nach Expression der SEQ ID NO:1 erhalten wurde, eine DNA-abhängige DNA Polymerase-Aktivität aufweist.

### Beispiel 6: Thermostabilität der Thermococcus pacificus DNA Polymerase

Die Thermostabilität der Polymerase wurde mit Hilfe des in Beispiel 6 beschriebenen Primerextensionstests überprüft. Hierzu wurden in Abwandlung des zuvor beschriebenen Assays je 1 unit der Polymerasepräparation für unterschiedliche Zeitpunkte bei 95°C vorinkubiert (0 min, 5 min, 10 min, 15 min, 30 min, 60 min und 90 min). Anschließend wurde mit diesen verschieden vorbehandelten Polymerasepräparationen der Assay wie in Beispiel 5 beschrieben durchgeführt. In Abwandlung zu den in Beispiel 5 beschriebenen Assays wurde als 1x Reaktionspuffer der Puffer der *Pfu* DNA Polymerase verwendet (Stratagene, Heidelberg). Die Ergebnisse sind in Abbildung 1 dargestellt. Zwei Kontrollreaktionen wurden ebenfalls mitgeführt, die keine DNA Polymerase enthielten. Die Ergebnisse zeigen deutlich, daß die Polymerase auch nach 90 min Inkubation keinerlei Aktivitätsverlust zeigt, d.h. eine äußerst hohe Thermostabilität aufweist (zum Vergleich: *Taq* DNA Polymerase verliert nach 60 min inkubation bei 94°C ca 50% der Polymeraseaktivität).

### Beispiel 7: Nachweis der 3'-5' Exonukleaseaktivität der Thermococcus pacificus DNA Polymerase

Mit dem im folgenden beschriebenen Test sollte überprüft werden, inwieweit die *Thermococcus pacficus* DNA Polymerase eine 3'-5' Exonukleaseaktivität aufweist. Die Fehlerrate mit der eine DNA Polymerase während der Replikation der chromosomalen DNA die parentale DNA dupliziert, ist von mehreren Faktoren abhängig: Zum einen ist die Auswahl der zur Ausgangsnukleinsäure komplementären Base entscheidend; inwieweit an ein falsch eingebautes Nukleotid ein weiteres Nukleotid von der Polymerase angehängt werden kann, und Inwieweit die Polymerase über eine 3'-5' Exonukleaseakrivität verfügt. Mit Hilfe einer solchen Exonukleaseaktivität können zuvor falsch eingebaute Nukleotide hydrolytisch abgespalten werden, so daß ein zuvor falsch eingebautes Nukleotid durch das korrekte komplementäre Nukleotid ersetzt werden kann. Diese Enzymaktivität wird auch als Korrekturlesefähigkeit einer Polymerase bezeichnet.

Der Nachweis der 3'-5' Exonukleaseaktivität wird durch den hydrolytischen Abbau einer DNA geführt. Zu diesem Zweck wurden folgende Substanzen gemischt: 1 µg DNA Größenmarker VI (Roche Biochemicals), optional 200 µM von jedem dNTP, 1 x *Pfu* DNA Polymerase Reaktionspuffer (Stratagene, Heidelberg) und 1 unit der *Thermococcus pacificus* DNA Polymerase. Reaktionen wurden mit und ohne Nukleotide angesetzt: In Abwesenheit von Nukleotiden überwlegt die Exonukleaseaktivität, wohingegen diese durch Zugabe von dNTPs gehemmt wird. Als Positivkontrolle wurde 1 unit *Pfu* DNA Polymerase eingesetzt (Stratagene, Heidelberg), die über eine 3'-5' Exonukleaseaktivität aufweist und als Negativkontrolle *Pfu* Exo minus DNA Polymerase (Stratagene, Heidelberg), deren Exonukleaseaktivität durch Punktmutagenese stark herabgesetzt ist. Die Reaktionen wurden für unterschiedliche Zeiten (5 min, 10 min, 30 min, 60 min und 90 min) bei 72°C inkubiert und anschließend auf einem 1%igen Agarosegel analysiert. Die Ergebnisse, die In Abbildung 2 dargestellt sind, machen deutlich, daß die *Thermococcus pacificus* DNA Polymerase eine vielfach höhere 3'-5' Exonukleaseaktivität im Vergleich zur *Pfu* DNA Polymerase aufweist.

### Beispiel 8: Nachweis der Fehlerkorrekturlesefähigkeit der Thermococcus pacificus DNA Polymerase unter PCR Bedingungen

Besondere Verwendung finden extrem thermostabile DNA Polymerasen mit Korrekturlesefähigkeit in PCR Reaktionen, die zur Herstellung von mutationsfreien DNA Fragmenten dienen sollen. Dies findet besondere Bedeutung für bestimmte molekularbiologische Anwendungen wie der Klonierung von fehlerfreien PCR Fragmenten für die Protelnexpression. DNA Polymerasen, die über eine 3'-5' Exonukleaseaktivität verfügen, synthetisieren PCR Produkte mit einer bis zu 12-fach geringeren Fehlerrate im Vergleich zur *Taq* DNA Polymerase, dem Standardenzym für PCR Anwendungen.

Im vorliegenden Test wurde die Fähigkeit der *Thermococcus pacificus* DNA Polymerase zur Fehlerkorrektur unter PCR Bedingungen in einem Mismatch-Reparatur Assay überprüft. Dieser Assay wurde speziell zu diesem Zweck entwikkelt (U.S. Patent 5,491,086) und setzt sich aus einem Amplifikationsschritt und einem diagnostischen Restriktionsverdau zusammen. Für die PCR Reaktion werden entweder Primer (Wildtypprimer) verwendet, die vollkommen homolog zur Ziel-DNA Sequenz sind (*Taq* DNA Polymerase Gen) und deren 151 bp großes PCR Produkt sich mit dem *BamHI* Restriktionsenzym spalten läßt, wodurch ein 132 bp und 19 bp großes DNA Fragment generiert wird. Parallel hierzu wurden Reaktionen durchgeführt, deren Primermoleküle am 3'-Ende eine Basenfehlpaarung aufweisen. Wird während der PCR die falsche Base nicht korrigiert, ist die *BamHI* Schnittstelle zerstört und das PCR Produkt kann nicht in einem Restriktionsverdau mit dem Enzym *BamHI* gespalten werden. Ist die Polymerase hingegen in der Lage, falsch gepaarte Basen zu erkennen und hydrolytisch zu entfernen, kann die korrekte komplementäre Base eingebaut werden und das PCR Produkt liefert nach Restriktionsdau die erwarteten zwei DNA Fragmente. Die verwendeten Wildtyp-Primer haben die Sequenzen: 5'-GCACCCCGCTTGGGCAGAG-3' (SEQ ID NO: 6) und 5'-TCCCGCCCCTCCTGGAAGAC-3' (SEQ ID NO: 7). Alternativ wurden Primer eingesetzt, deren 3'-Ende eine C:A, C:T oder C:C Fehlpaarung aufwiesen. PCR Reaktionen wurden mit der Thermococcus *pacificus* DNA Polymerase, der Pfu DNA Polymerase (Stratagene, Heidelberg, Deutschland), die über eine Mismatch-Korrektur verfügt, und der *Pfu Exo minus* DNA Polymerase (Stratagene, Heidelberg, Deutschland), die diese Fehlerkorrektur nicht durchführen kann, durchgeführt. PCR Reaktionen enthielten 20 ng Plasmid pQE-31 (Qiagen GmbH, Hilden, Deutschland), das die *Taq* DNA Polymerasegen Zielsequenz enthielt, 1 unit der jeweiligen DNA Polymerase, 1x Pfu Reaktionspuffer, 200 µM von jedem dNTP und 1.5 µM jedes Primers. Das Reaktionsendvolumen betrug 50 µl. Die PCR Reaktionen wurden in einem MJ Research PTC-200 Thermocycler (Biozym, Hess. Oldendorf, Deutschland) durchgeführt. Das PCR Programm setzte sich aus einen initialen Denaturierungsschritt für 1 min bei 94°C, einem Denaturierungsschritt von 30 sec bei 94°C, einem Hybridisierungsschritt bei 62°C und einem Extensionsschritt bei 72°C für 1 min zusammen. PCR Produkte wurden mit dem QIAquick PCR Purification Kit aufgereinigt (QIAGEN GmbH, Hilden, Deutschland). Identische Mengen an PCR Produkt wurden pro 100 ng PCR Produkt mit einer unit *BamHI* für 90 min bei 37°C verdaut. Anschließend wurden die so behandelten PCR Produkte auf einem 4%igen Metaphor Agarose Gel (Biozym, Hess. Oldendorf, Deutschland) analysiert. Die Ergebnisse sind in Abbildung 3 wiedergegeben. Die Ergebnisse zeigen, daß die *Pfu Exo minus* DNA Polymerase wie erwartet nicht in der Lage ist, den Mismatch zu reparieren, d.h. das mit mutierten Primem erzeugte PCR Produkt kann von dem Restriktionsenzym *BamHI* nicht gespalten werden. Im Gegensatz hierzu ist die nicht mutierte Pfu DNA Polymerase fähig, die Basenfehlpaarung zu korrigieren. Die *Thermococcus pacificus* DNA Polymerase ist zum einen in der Lage, unter PCR Bedingungen das gewünschte PCR Produkt zu synthetisieren, und kann darüber hinaus falsch gepaarte Basen unter PCR Bedingungen erkennen, diese hydrolytisch entfernen und an deren Stelle das korrekte komplementäre Nukleotid einfügen. Somit ist gezeigt, daß die *Thermococcus pacificus* DNA Polymerase zur Durchführung von sogenannten High Fidelity PCR Reaktionen geeignet ist.

## Patentansprüche

1. DNA-Molekül, das für eine thermostabile DNA-Polymerase mit 5'-3'-DNA-Polymerase-Aktivität kodiert, und das
(a) die Sequenz SEQ ID NO: 1 oder die dazu komplementäre Sequenz enthält oder
(b) eine Sequenz enthält, die für einen zusammenhängenden Abschnitt von wenigstens 200 Aminosäuren der Sequenz SEQ ID NO: 2 kodiert, oder
(c) eine Sequenz enthält, die der SEQ ID NO: 1 oder der dazu komplementären Sequenz so ähnlich ist, dass das DNA Molekül unter stringenten Bedingungen, bei denen zwei DNA-Moleküle in einem Hybridisierungsexperiment hybridisieren, wenn sie über die gesamte Länge eine Sequenzidentität von 98% oder mehr aufweisen, miteinander hybridisieren,
(d) mit dem Proviso, das nicht das Polynukleotid umfasst ist, welches für eine Polymerase kodiert, bestehend aus (i) Aminosäuren 271 bis 832 von *Taq* Polymerase (SEQ ID NO. 7 in WO 01/61015), verbunden mit (ii) Aminosäuren 1 bis 395 von *Tpac* (SEQ ID NO. 16 in WO 01/61015).

2. DNA-Molekül nach Anspruch 1, **dadurch gekennzeichnet, dass** die thermostabile DNA-Polymerase 3'-5'-Exonuklease-Aktivität hat.

3. Vektor, enthaltend ein DNA-Molekül gemäß einem der Ansprüche 1 oder 2.

4. Vektor nach Anspruch 3, **dadurch gekennzeichnet, dass** es ein Expressionsvektor ist.

5. Vektor nach Anspruch 4, enthaltend die folgenden Merkmale:
(a) einen oder mehrere Promotoren;
(b) mindestens einen Operator, der zu Steigerung oder Unterdrückung der Genexpression verwendet werden kann;
(c) Terminationssequenzen für Transkription und Translation.

6. Polypeptid mit 5'-3'-DNA-Polymerase-Aktivität, das durch ein DNA-Molekül gemäß einem der Ansprüche 1 oder 2 kodiert wird.

7. Polypeptid nach Anspruch 6, **dadurch gekennzeichnet, dass** es 3'-5'-Exonuklease-Aktivität hat.

8. Polypeptid mit 5'-3'-DNA-Polymerase-Aktivität, **dadurch gekennzeichnet, dass** es eine Sequenz enthält,
(a) die einen zusammenhängenden Abschnitt von wenigstens 200 Aminosäuren der Sequenz SEQ ID NO: 2 darstellt,
(b) mit dem Proviso, das nicht das Polypeptid umfasst ist, mit den Aminosäuren 271 bis 832 von *Taq* Polymerase (SEQ ID NO. 7 in WO 01/61015), verbunden mit den Aminosäuren 1 bis 395 von *Tpac* (SEQ ID NO. 16 in WO 01/61015).

9. Polypeptid nach Anspruch 8, das die Sequenz SEQ ID NO: 2 enthält.

10. Wirtszelle, enthaltend einen Vektor gemäß einem der Ansprüche 3 bis 5.

11. Wirtszelle nach Anspruch 10, **dadurch gekennzeichnet, dass** sie Escheria coli ist.

12. Verfahren zur Herstellung eines Polypeptids gemäß einem der Ansprüche 6 bis 9, **dadurch gekennzeichnet, dass**
(a) Wirtszellen gemäß einem der Ansprüche 10 oder 11 einem geeigneten Medium kultiviert werden; und
(b) das Polypeptid aus dem Medium oder den Wirtszellen isoliert wird.

13. Verwendung eines Polypeptids mit 5'-3'-DNA-Polymerase-Aktivität gemäß Anspruch 6 bis 9 zur Polymerisation von Nukleinsäure.

14. Verwendung nach Anspruch 13, **dadurch gekennzeichnet, dass** die Polymerisation durch Polymerase-Kettenreaktion (PCR) bewirkt wird.

15. Kit zur Verwendung für die Polymerisation von Nukleinsäure, in separaten Behältern enthaltend:
(a) ein Polypeptid gemäß einem der Ansprüche 6 bis 9; und
(b) einen Reaktionspuffer für die Polymerisationsreaktion; und optional
(c) dATP, dGTP, dCTP, und dTTP, entweder als Gemisch oder in jeweils individuellen Behältern.

## Claims

1. A DNA molecule coding for a thermostable DNA polymerase with 5'-3' DNA polymerase activity,
(a) wherein the DNA molecule comprises the sequence of SEQ ID NO. 1 or a sequence complementary thereto; or
(b) wherein the DNA molecule comprises a sequence encoding a continuous region of at least 200 amino acids according to SEQ ID NO. 2; or
(c) wherein the DNA molecule comprises a sequence hybridizing to a DNA molecule having the sequence of SEQ ID NO. 1 under stringent conditions under which two DNA molecules of at least 98% sequence identity over the whole length hybridize; and
(d) with the proviso that the a polynucleotide coding for a polymerase consisting of (i) amino acids 271 to 832 of *Taq* polymerase (SEQ ID NO. 7 of WO 01/61015), in combination with (ii) amino acids 1 to 395 of *Tpac* (SEQ ID NO. 16 of WO 01/61015) is not comprised.

2. The DNA molecule according to claim 1, wherein the DNA polymerase comprises 3'-5' exonuclease activity.

3. A vector comprising a DNA molecule according to claim 1 or 2.

4. The vector according to claim 3, wherein the vector is an expression vector.

5. The vector according to claim 4 comprising the following features:
(a) one or more promoter sequences;
(b) at least one operator sequence suited for inducing or repressing gene expression;
(c) terminator sequences for terminating transcription and translation.

6. A polypeptide having 5'-3' polymerase activity encoded by DNA molecule according to claims 1 or 2.

7. The polypeptide according to claim 6, wherein the polypeptide comprises 3'-5' exonuclease activity.

8. A polypeptide having 5'-3' DNA polymerase activity, wherein the polypeptide comprises
(a) a continuous region of at least 200 amino acids according to SEQ ID NO. 2; and
(b) with the proviso that the a polypeptide consisting of (i) amino acids 271 to 832 of *Taq* polymerase (SEQ ID NO. 7 of WO 01/61015), in combination with (ii) amino acids 1 to 395 of *Tpac* (SEQ ID NO. 16 of WO 01/61015) is not comprised.

9. The polypeptide according to claim 8, wherein the polypeptide comprises the sequence of SEQ ID NO. 2.

10. A host cell comprising a vector according to any one of claims 3 to 5.

11. The host cell according to claim 10, wherein the host cell is a *Escherichia coli* cell.

12. A method for producing a polypeptide according to any one of claims 6 to 9,
(a) wherein a host cell according to claim 10 or 11 is cultivated in suited medium, and
(b) wherein the polypeptide is isolated from the medium or the host cell.

13. Use of a polypeptide with 5'-3' DNA polymerase activity according to any one of claims 6 to 9 for polymerisation of nucleic acids.

14. The use according to claim 13, wherein the polymerisation of nucleic acids causes a polymerase chain reaction (PCR).

15. A kit for use in polymerizing nucleic acids comprising in separated container the following:
(a) a polypeptide according to any one of claims 6 to 9; and
(b) a reaction buffer for polymerisation reactions; and optionally
(c) dATP, dGTP, dCTP, and dTTP as a mixture or each in a separated container.

## Revendications

1. Molécule d'ADN qui code une ADN polymérase thermostable présentant une activité d'ADN polymérase en 5'-3', et qui contient
(a) la séquence SEQ ID N° : 1 ou la séquence complémentaire de celle-ci, ou
(b) une séquence qui code un segment continu d'au moins 200 acides aminés de SEQ ID N° : 2, ou
(c) une séquence qui est similaire à SEQ ID N° : 1 ou à la séquence complémentaire de celle-ci de telle sorte que la molécule d'ADN s'hybride dans des conditions stringentes dans lesquelles deux molécules d'ADN s'hybrident dans une expérience d'hybridation lorsqu'elles présentent une identité de séquence sur la longueur totale de 98 % ou plus,
(d) à l'exclusion du polynucléotide qui code pour une polymérase constituée (i) des acides aminés 271 à 832 de la polymérase *Taq* (SEQ ID N° : 7 dans WO 01/61015), liés (ii) aux acides aminés 1 à 395 de *Tpac* (SEQ ID N° : 16 dans WO 01/61015).

2. Molécule d'ADN selon la revendication 1, **caractérisée en ce que** l'ADN polymérase thermostable a une activité d'exonucléase en 3'-5'.

3. Vecteur contenant une molécule d'ADN selon l'une des revendications 1 ou 2.

4. Vecteur selon la revendication 3, **caractérisé en ce qu'**il est un vecteur d'expression.

5. Vecteur selon la revendication 4, contenant les caractéristiques suivantes :
(a) un ou plusieurs promoteurs ;
(b) au moins un opérateur qui peut être utilisé pour augmenter ou pour inhiber l'expression de gènes ;
(c) des séquences de terminaison de transcription et de traduction.

6. Polypeptide présentant une activité d'ADN polymérase en 5'-3' qui est codé par une molécule d'ADN selon l'une des revendications 1 ou 2.

7. Polypeptide selon la revendication 6, **caractérisé en ce qu'**il a une activité d'exonucléase en 3'-5'.

8. Polypeptide présentant une activité d'ADN polymérase en 5'-3', **caractérisé en ce qu'**il contient une séquence
(a) qui est un segment continu d'au moins 200 acides aminés de la séquence SEQ ID N° : 2,
(b) à l'exclusion du polypeptide comprenant les acides aminés 271 à 832 de la polymérase *Taq* (SEQ ID N° : 7 dans WO 01/61015), liés aux acides aminés 1 à 395 de *Tpac* (SEQ ID N° : 16 dans WO 01/61015).

9. Polypeptide selon la revendication 8, qui contient la séquence SEQ ID N° : 2.

10. Cellule hôte contenant un vecteur selon l'une des revendications 3 à 5.

11. Cellule hôte selon la revendication 10, **caractérisé en ce qu'**il s'agit de *Escherichia coli.*

12. Procédé de production d'un polypeptide selon l'une des revendications 6 à 9, **caractérisé en ce que**
(a) des cellules hôtes selon l'une des revendications 10 ou 11, sont cultivées dans un milieu approprié ; et
(b) le polypeptide est isolé à partir du milieu ou des cellules hôtes.

13. Utilisation d'un polypeptide présentant l'activité d'ADN polymérase en 5'-3' selon les revendications 6 à 9 pour la polymérisation d'acide nucléique.

14. Utilisation selon la revendication 13, **caractérisé en ce que** la polymérisation est une amplification en chaîne par polymérase (PCR).

15. Kit pour la polymérisation d'acide nucléique contenant, dans des conteneurs séparés :
(a) un polypeptide selon l'une des revendications 6 à 9 ; et
(b) un tampon réactionnel pour la réaction de polymérisation ; et éventuellement,
(c) du dATP, dGTP, dCTP et dTTP, sous forme de mélange ou dans des conteneurs individuels.


### SEQUENZPROTOKOLL

<110> Qiagen GmbH
<120> Thermostabile Polymerase aus Thermococcus pacificus
<130> F 2260 PCT
<140>
   <141>
<160> 7
<170> PatentIn Ver. 2.1
<210> 1
   <211> 2322
   <212> DNA
   <213> *Thermococcus pacificus*
<400> 1
   atgatcctcg atgccgacta cattaccgaa gacggaaagc ccgtcatcag gatattcagg 60
   aaggagaagg gcgagtttaa gatcgagtac gacaggaatt ttgagcccta cttctatgcc 120
   ctcctgaagg acgattctgc gattgaagac gtcaaaaaga taaccgcgga gaggcacggc 180
   actaccgtta gggtcgtcag ggccgagaag gtgaagaaga agttcctcgg caggccgata 240
   gaggtctgga agctctactt caccccaccc caggacgttc ccgcaatcag agacaaaata 300
   agggaacatc cagccgttgt ggacatctac gagtacgaca tccccttcgc gaagcgctac 360
   ctcatagaca agggcttaat cccgatggag ggcgatgaag aacttaagat gctcgccttc 420
   gacatcgaga cgctctacca cgagggcgag gagttcgccg aagggcccat cctgatgata 480
   agctacgccg acgaggaagg ggcgcgcgtt attacctgga agaatatcga cctcccctac 540
   gttgacgtcg tttccactga gaaggagatg ataaagcgct tcctcagggt catcaaggag 600
   aaggatcccg acgtgctaat aacatacaac ggcgataact tcgacttcgc ctacctcaag 660
   aagcgctccg agaagctcgg cgtcaagttc atcctcggga gggacggcag tgagccgaaa 720
   attcagagga tgggcgaccg ctttgcagtg gaggtcaagg gaaggattca cttcgacctc 780
   taccccgtca taaggaggac gattaacctg cccacttaca cccttgaggc agtatatgaa 840
   gccatctttg gacagccaaa ggagaaggtc tacgcggaag agataacgca ggcctgggag 900
   acgggcgagg gattagaaag ggtggcccgc tactcgatgg aagatgctaa ggtaacctac 960
   gagctcggaa aagagttctt cccgatggag gcccagctct cgcgtctcgt aggccagagc 1020
   ctctgggatg tctcgcgctc gagtaccgga aacctcgtcg agtggttttt gctgaggaag 1080
   gcctacgaga ggaacgagct tgcaccaaac aagccagacg agaaggaact ggccaggaga 1140
   agggagagct acgcgggtgg atacgtcaag gagcctgaga aaggactgtg ggaaaacata 1200
   gtctacttag attttcgctc tctctacccc tctataatta ttacacacaa cgtctcccca 1260
   gatacgctca acagggaggg ctgtaaggag tacgacgtgg ctcctcaggt gggacaccgc 1320
   ttctgcaagg acttccccgg cttcattccg agcctcctcg gagacctttt ggaggagagg 1380
   cagaagataa agaagaacat gaaggccacg atagacccga tcgagaagaa gctccttgat 1440
   tacaggcaac gggccattaa aatccttgct aatagtttct acggttacta cggctacgca 1500
   aaggcccgct ggtactgcag ggagtgcgcc gagagcgtga cagcttgggg caggcagtac 1560
   atcgagacca cgataaggga aatagaggat aaatttggct ttaaagtgct ttacgcagac 1620
   accgacgggt ttttcgcgac aatcccagga gctgatgccg aaaccgtcaa aaagaagtcc 1680
   aaggagttcc tcaagtatat taacgccaag ctgcccggcc tgctcgagct ggagtacgag 1740
   ggcttctaca agcgcggttt cttcgtgacg aagaagaagt acgcggttat agacgaggaa 1800
   gacaagataa cgacgcgcgg gcttgaaata gtcaggcgtg actggagcga gatagcaaag 1860
   gagacgcagg cgagggttct tgaggcgata ctcaagcacg gtgacgttga ggaagctgta 1920
   aagattgtca aagaggtgac ggaaaagctc agcaagtacg aagttccacc agagaagctt 1980
   gtcatccacg agcagataac tcgcgacctg aaggactaca aggccaccgg cccgcatgtg 2040
   gctgttgcaa agcgcctcgc cgcgagggga ataaaaatcc ggcccgggac ggtcataagc 2100
   tacatcgtgc tcaaaggctc tggaaggatt ggggacaggg ctataccgtt cgacgagttc 2160
   gatccgacaa agcacaagta cgatgcggaa tactacatcg agaaccaggt tctgccggcg 2220
   gtggagagaa ttctgagggc ctttggctac cgtaaggagg acctacgcta ccagaagacg 2280
   cggcaggttg gcttgggggc gtggctaaaa cctaagacat ga 2322
<210> 2
   <211> 773
   <212> PRT
   <213> *Thermococcus pacificus*
<900> 2
<210> 3
   <211> 21
   <212> DNA
   <213> Künstliche Sequenz
<220>
   <223> Beschreibung der künstlichen Sequenz:Primer
<400> 3
   atgatcctcg atgccgacta c 21
<210> 4
   <211> 24
   <212> DNA
   <213> Künstliche Sequenz
<220>
   <223> Beschreibung der künstliche Sequenz:Primer
<400> 4
   tcatgtctta ggttttagcc acgc 24
<210> 5
   <211> 30
   <212> DNA
   <213> Künstliche Sequenz
<220>
   <223> Beschreibung der künstlichen Sequenz:Primer
<400> 5
   tttcccagtc acgacgttgt aaaacgacgg 30
<210> 6
   <211> 19
   <212> DNA
   <213> Künstliche Sequenz
<220>
   <223> Beschreibung der künstlichen Sequenz:Primer
<400> 6
   gcaccccgct tgggcagag 19
<210> 7
   <211> 20
   <212> DNA
   <213> Künstliche Sequenz
<220>
   <223> Beschreibung der künstlichen Sequenz:Primer
<400> 7
   tcccgcccct cctggaagac 20